(19) **Europäisches Patentamt / European Patent Office / Office européen des brevets**

(11) **EP 4 636 077 A1**

(12) **EUROPEAN PATENT APPLICATION**
published in accordance with Art. 153(4) EPC

(43) Date of publication:
**22.10.2025 Bulletin 2025/43**

(21) Application number: **23903508.2**

(22) Date of filing: **12.12.2023**

(51) International Patent Classification (IPC):
*C12N 5/077* (2010.01)    *C12N 5/078* (2010.01)
*C12N 5/0775* (2010.01)

(52) Cooperative Patent Classification (CPC):
**C12N 5/06**

(86) International application number:
**PCT/JP2023/044432**

(87) International publication number:
**WO 2024/128224 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **12.12.2022   JP 2022197877**

(71) Applicant: **Juntendo Educational Foundation Tokyo 113-8421 (JP)**

(72) Inventors:
• **TANAKA, Rica**
  **Tokyo 113-8421 (JP)**
• **FURUKAWA, Satomi**
  **Tokyo 113-8421 (JP)**

(74) Representative: **Mewburn Ellis LLP**
  **Aurora Building**
  **Counterslip**
  **Bristol BS1 6BX (GB)**

(54) **COMPOSITION FOR AMPLIFYING ABILITY OF MESENCHYMAL STEM CELLS TO DIFFERENTIATE INTO ADIPOCYTES**

(57)   The present invention relates to a composition and a method for amplifying ability of a mesenchymal stem cell to differentiate into an adipocyte. The composition of the present invention contains in vitro amplified and cultured mononuclear cell fraction, and is co-cultured with a mesenchymal stem cell. The method of the present invention is a method for amplifying ability of a mesenchymal stem cell to differentiate into an adipocyte comprising co-culturing in vitro amplified and cultured mononuclear cell fraction with a mesenchymal stem cell.

EP 4 636 077 A1

**Description**

TECHNICAL FIELD

The present invention relates to a composition for amplifying ability of a mesenchymal stem cell to differentiate into an adipocyte and a method for amplifying ability of a mesenchymal stem cell to differentiate into an adipocyte.

BACKGROUND ART

In recent years, treatments using mesenchymal stem cells have been studied in many diseases. The mesenchymal stem cells are somatic stem cells derived from mesodermal tissue (mesenchyme), and have ability to differentiate into cells belonging to the mesenchymal cell. Depending on the tissue from which the mesenchymal stem cells are collected, they are called adipose-derived mesenchymal stem cells, bone marrow-derived mesenchymal stem cells, and the like. Mesenchymal stem cells are known to have pluripotency to differentiate into various somatic cells, such as bone, blood vessel, and cardiac muscle cells, and also secrete cytokines and have an immunosuppressive effect, an anti-inflammatory effect, and an angiogenic effect. In addition, mesenchymal stem cells can be grafted not only autologously but also allogeneically, due to their high immunotolerance capability.

Due to these characteristics, mesenchymal stem cells are pluripotent stem cells with great potential in regenerative medicine. Up to the present time, numerous clinical studies thereon have been conducted for various diseases, and they are expected to be used to treat a wide variety of diseases.

Breast cancer is cancer with the highest morbidity in women, and its morbidity tends to increase year by year. Approximately 40% of patients with breast cancer require mastectomy (2017). The mastectomy brings great suffering, especially mentally. Fat grafting is performed in the field of soft tissue reconstruction, such as breast reconstruction after breast cancer surgery, for the purpose of increasing the volume and restoring the contour. This technique uses abundantly available adipose tissue as an autologous filler, which can achieve a natural feel, and is an inexpensive treatment with a low infection rate. Thus, adipose tissue is superior to exogenous materials. However, during the process of collecting fat by liposuction, adipocytes are cut off from original blood supply. As a result, the graft after its injection undergoes temporary ischemia, which causes partial necrosis and graft volume loss (NPL 1). Fat grafting and its long-term clinical application have been drawing increasing attention (NPL 2). However, a satisfactory solution to such volume loss due to ischemia still remains to be developed. In order for fat grafting to achieve satisfactory results, a secondary treatment is often required. It is demanded to develop a fat grafting technique which achieves higher fat regeneration and vascular regeneration capabilities.

As a cell-based therapy for enhancing fat grafting, adipose-derived mesenchymal stem cells (ASCs) have been studied (NPL 13). A therapy in which ASCs are supplemented when adipocytes are grafted (ASC-supplemented fat grafting method) is promising, but requires further development in order to obtain consistent clinical results (NPLs 14 and 15). ASCs have been observed to release proangiogenic cytokines (NPL 16) and differentiate into vascular endothelium in vitro (NPL 17).

The present inventors revealed in WO2019/146131 (PTL 3) that a composition containing in vitro amplified and cultured mononuclear cell fraction has an effect to amplify an effect of mesenchymal stem cells. Specifically, it was found that supplementing in vitro amplified and cultured mononuclear cell fraction to a treatment using mesenchymal stem cells safely and effectively amplified effects of the treatment by stimulating angiogenesis and exerting an anti-inflammatory effect and an anti-immune effect, using two models: fat grafting and limb ischemia. These effects to amplify effects of the mesenchymal stem cells were observed when the mesenchymal stem cells were co-grafted with in vitro amplified and cultured mononuclear cell fraction. WO2019/146131 describes a composition for amplifying an effect of a treatment using a mesenchymal stem cell, which contains in vitro amplified and cultured mononuclear cell fraction, and a method for amplifying an effect of a treatment using a mesenchymal stem cell, which includes using a composition containing in vitro amplified and cultured mononuclear cell fraction together with the mesenchymal stem cell or a composition containing the mesenchymal cell.

WO2019/146131 describes that, when mesenchymal stem cells are co-grafted with in vitro amplified and cultured mononuclear cell fraction, the angiogenesis capability of the mesenchymal stem cells is enhanced. This literature describes that effects on fat grafting are also based on the enhancement of the angiogenesis capability. WO2019/146131 does not mention at all to differentiation of adipose-derived mesenchymal stem cells (ASCs) into adipocytes.

CITATION LIST

PATENT LITERATURE

PTL 1: WO2006/090882
PTL 2: WO2014/0561154
PTL 3: WO2019/146131
PTL 4: WO2021/131261

NON PATENT LITERATURE

NPL 1: Plast Reconstr Surg. 2013 Feb; 131(2): 185-191
NPL 2: Langenbecks Arch Klin Chir Ver Dtsch Z Chir. 1893; 22: 66-69
NPL 3: Otolaryngol Head Neck Surg. 1990 Apr; 102(4): 314-321
NPL 4: Ann Plast Surg. 2005 Jul; 55(1): 63-68
NPL 5: Exp Biol Med (Maywood). 2005 Nov; 230(10): 742-748
NPL 6: Dermatol Surg. 2006 Dec; 32(12): 1437-1443
NPL 7: Plast Reconstr Surg Glob Open. 2013 Oct 7; 1(6): e40
NPL 8: Plast Reconstr Surg. 2012 May; 129(5): 1081-1092
NPL 9: Plast Reconstr Surg. 2015 Jun; 135(6): 1607-1617
NPL 10: J Am Coll Cardiol. 2003 Dec 17; 42(12): 2073-2080
NPL 11: Plast Reconstr Surg. 2008 Jun; 121(6): 1929-1942
NPL 12: Stem Cells Transl Med. 2012 Feb; 1(2): 160-171
NPL 13: Mol Biol Cell. 2002 Dec; 13(12): 4279-4295
NPL 14: J Plast Reconstr Aesthet Surg. 2013 Nov; 66(11): 1494-1503
NPL 15: Aesthet Surg J. 2017 Jul 1; 37(suppl_3): S46-58
NPL 16: Stem Cells. 2009 Jan; 27(1): 266-274
NPL 17: Circulation. 2004 Feb 10; 109(5): 656-663
NPL 18: PLoS One. 2012; 7(9): e45621
NPL 19: Lancet. 2013 Sep 28; 382(9898): 1113-1120
NPL 20: Diabetes. 2013 Sep; 62(9): 3207-3217
NPL 21: J Am Heart Assoc. 2014 Jun 25; 3(3): e000743
NPL 22: Japanese Journal of Thrombosis and Hemostasis 2014;25(5):593-602

SUMMARY OF INVENTION

TECHNICAL PROBLEM

Although researches on fat grafting using mesenchymal stem cells have been progressing, further researches and developments are needed in order to achieve better effects.

SOLUTION TO PROBLEM

The present inventors conducted diligent studies in order to solve the problem described above, and as a result, they found that in vitro amplified and cultured mononuclear cell fraction amplified ability of mesenchymal stem cells to differentiate into adipocytes, and arrived at the present invention. The present invention non-limitingly includes the following embodiments.

[Embodiment 1] A composition for amplifying ability of a mesenchymal stem cell to differentiate into an adipocyte, wherein the composition contains in vitro amplified and cultured mononuclear cell fraction and is co-cultured with a mesenchymal stem cell.

[Embodiment 2] The composition according to embodiment 1, wherein the composition is separated from the mesenchymal stem cell during differentiation of the mesenchymal stem cell into an adipocyte.

[Embodiment 3] The composition according to embodiment 1, wherein the in vitro amplified and cultured mononuclear cell fraction is co-cultured with a mesenchymal stem cell for one to seven days.

[Embodiment 4] The composition according to embodiment 1 or 2, wherein the mononuclear cell fraction is a mononuclear cell fraction derived from bone marrow, peripheral blood, or umbilical cord blood.

[Embodiment 5] The composition according to embodiment 1 or 2, wherein the in vitro amplified and cultured mononuclear cell fraction is a group of cells rich in endothelial progenitor cells or anti-inflammatory cells or immunotolerance-inducing cells.

[Embodiment 6] The composition according to embodiment 1 or 2, wherein the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell.

[Embodiment 7] A method for amplifying ability of a mesenchymal stem cell to differentiate into an adipocyte, the method comprising co-culturing in vitro amplified and cultured mononuclear cell fraction with a mesenchymal stem cell.

[Embodiment 8] The method according to embodiment 7, further including separating the in vitro amplified and cultured mononuclear cell fraction from a mesenchymal stem cell.

ADVANTAGEOUS EFFECTS OF INVENTION

The mesenchymal stem cell co-cultured with the in vitro amplified and cultured mononuclear cell fraction according to the present invention has higher ability to differentiate into an adipocyte, as compared to a mesenchymal stem cell without the co-culturing. The mesenchymal stem cell with the amplified ability to differentiate into an adipocyte enhances the efficiency of fat grafting using the mesenchymal stem cell.

BRIEF DESCRIPTION OF DRAWINGS

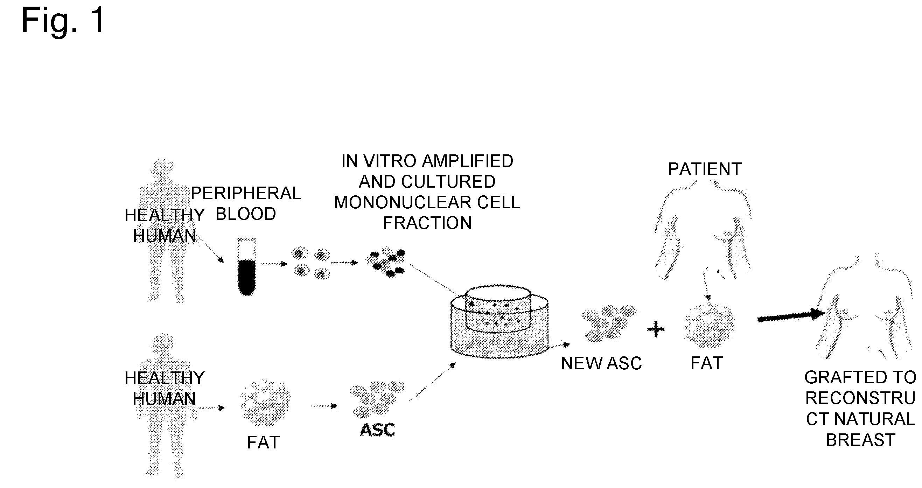

[Fig. 1] Fig. 1 is a schematic diagram illustrating embodiments of preparation of mesenchymal stem cells and fat grafting using the mesenchymal stem cells according to the present invention.

[Fig. 2] Fig. 2 illustrates results of mRNA expression analysis of ASCs co-cultured with RE01 cells in an adipogenic differentiation inducing medium for three days. Fig. 2A illustrates the results for FABP4, and Fig. 2B illustrates the results for PPAR$\gamma$.

[Fig. 3] Fig. 5 illustrates results of analyzing capability of the ASCs to induce adipogenic differentiation by staining lipid droplets with Oil Red O using ASCs cultured with RE01 cells in an adipogenic differentiation inducing medium for two weeks.

[Fig. 4] Fig. 4 illustrates results of mRNA expression analysis of ASCs which were co-cultured with RE01 cells for three days and then, after removal of the RE01 cells, were cultured in an adipogenic differentiation inducing medium for three days.

[Fig. 5] Fig. 5 illustrates results of staining lipid droplets with Oil Red O using ASCs which were co-cultured with RE01 cells for three days and then, after removal of the RE01 cells, were cultured in an adipogenic differentiation inducing medium for two weeks.

DESCRIPTION OF EMBODIMENTS

Embodiments for implementing the present invention include the following forms.

As mentioned herein, the term "in one embodiment" means that the invention is not limited to that embodiment, that is, the embodiment is non-limiting. The terms "non-limitingly" and "in one embodiment" can be used herein synonymously.

1. Composition for Amplifying Ability of Mesenchymal Stem Cell to Differentiate into Adipocyte

The present invention relates to a composition for amplifying ability of a mesenchymal stem cell to differentiate into an adipocyte. In one embodiment, the composition of the present invention is a composition which contains in vitro amplified and cultured mononuclear cell fraction and is co-cultured with a mesenchymal stem cell.

The term "mononuclear cell fraction" collectively refers to cells having a round nucleus which are contained in peripheral blood, bone marrow, umbilical cord blood, or the like obtained from an organism, and includes lymphocytes, monocytes, macrophages, endothelial progenitor cells, hematopoietic stem cells, and the like. The mononuclear cells further include CD34-positive and/or CD133-positive cells. They are obtained by collecting bone marrow, umbilical cord blood, or peripheral blood from an animal and subjecting it to a density gradient centrifugation method, for example, to extract the fraction.

In one embodiment, the mononuclear cell fraction is a mononuclear cell fraction derived from bone marrow, peripheral blood, or umbilical cord blood.

In one embodiment, the in vitro amplified and cultured mononuclear cell fraction is a group of cells rich in endothelial progenitor cells or anti-inflammatory cells or immunotolerance-inducing cells. This group of cells includes endothelial progenitor cells, anti-inflammatory macrophages, T-lymphocyte subsets, and regulatory T-cells.

**EP 4 636 077 A1**

The term "endothelial progenitor cell (EPC)" particularly refers to CD34-positive and/or CD133-positive cells among the mononuclear cell fraction. It also includes differentiated EPC colony-forming cells. Endothelial progenitor cells are known as cells capable of differentiating into blood vessels, and are believed to be capable of repairing or supplementing a damaged blood vessel.

In one embodiment, a group of "anti-inflammatory cells or immunotolerance-inducing cells" are a group of cells rich in endothelial progenitor cells, M2 macrophages, T-lymphocyte subsets, or regulatory T-cells. These cells are believed to have an anti-inflammatory or immunotolerance-inducing function (NPL 22).

The term "in vitro amplification (Quality and Quantity) (QQ)" refers to culturing stem cells collected from an organism in vitro in a serum-free medium or a serum medium containing a factor, such as a stem cell growth factor and interleukin, for a certain period for modification and amplification. The term "modification and amplification" means increasing the number of stem cells, and/or enhancing their function.

The method for amplifying and in vitro culturing the mononuclear cell fraction is not particularly limited, and any known method can be used. In one embodiment, stem cells are cultured in a serum-free medium or a serum medium containing one or two or more factors selected from the group consisting of a stem cell factor, interleukin-6, an FMS-like tyrosine kinase 3 ligand, thrombopoietin, and a vascular endothelial growth factor. Non-limitingly, the stem cells are cultured in a serum-free medium or a serum medium containing one or more, two or more, three or more, four or more, or five or more factors selected from the group consisting of a stem cell factor, interleukin-6, an FMS-like tyrosine kinase 3 ligand, thrombopoietin, and a vascular endothelial growth factor.

In one embodiment, the stem cells are cultured in a serum-free medium or a serum medium containing one or two or more factors selected from the group consisting of an FMS-like tyrosine kinase 3 ligand, thrombopoietin, and a vascular endothelial growth factor.

For example, PTL 2 describes a group of cells obtained by culturing mononuclear cells derived from bone marrow, umbilical cord blood, or peripheral blood in a serum-free medium containing a stem cell factor, interleukin-6, an FMS-like tyrosine kinase 3 ligand, thrombopoietin, and a vascular endothelial growth factor. PTL 4 describes a group of cells obtained by culturing mononuclear cells derived from bone marrow, umbilical cord blood, or peripheral blood in a medium containing serum and four or less factors selected from the group consisting of a stem cell factor, interleukin-6, an FMS-like tyrosine kinase 3 ligand, thrombopoietin, and a vascular endothelial growth factor. The in vitro amplified and cultured mononuclear cell fraction preferably has a characteristic of being rich in endothelial progenitor cells, and anti-inflammatory cells or immunotolerance-inducing cells. In one embodiment, it also includes anti-inflammatory M2 macrophages, T-lymphocyte subsets, and regulatory T-cells.

For example, PTL 4 and NPLs 12, 20, and 21 disclose specific methods for amplifying and in vitro culturing endothelial progenitor cells. For example, a medium supplemented with a VEGF, an SCF, an Flt-3 ligand, TPO, IL-6, and 1% antibiotics can be used. In the Examples in the specification of the present application, cells were cultured for one week in an IMDM medium (including GlutaMAX, Gibco) supplemented with 50 ng/mL of VEGF, 100 ng/mL of FLT-3 ligand, 20 ng/mL of TPO, 0.5% FBS (fetal bovine serum), 0.5% HSA, and 1% antibiotics.

Alternatively, as the method for amplifying and in vitro culturing the mononuclear cell fraction, the method described in WO2006/090882 may be used. This method includes incubating endothelial progenitor cells in a serum-free medium containing one or two or more factors selected from the group consisting of a stem cell growth factor, interleukin-6, FMS-like tyrosine kinase 3, and thrombopoietin.

The composition containing the in vitro amplified and cultured mononuclear cell fraction is co-cultured with a mesenchymal stem cell. In one embodiment, the co-culturing is performed in vitro or ex vivo.

The term "mesenchymal stem cell" refers to somatic stem cells derived from mesodermal tissue (mesenchyme), which have ability to differentiate into cells belonging to the mesenchymal cell. Depending on the tissue from which the mesenchymal stem cells are collected, they are called adipose-derived mesenchymal stem cells (ASCs), bone marrow-derived mesenchymal stem cells, and the like. Mesenchymal stem cells are included in stromal cells. For example, when induced for differentiation, bone marrow stromal cells become cells belonging to the mesenchymal system (bone cells, cardiac muscle cells, chondrocytes, tenocytes, adipocytes, and the like).

In one embodiment, the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell. In one embodiment, the mesenchymal stem cell is an adipose-derived mesenchymal stem cell.

In one embodiment, the composition containing the mesenchymal stem cell is a stromal vascular cell fraction (SVF) (also referred to as "group of stromal vascular cells"). The stromal vascular cell fraction is a group of cells obtained by treating adipose tissue with collagenase. The stromal vascular cell fraction is a generally heterogeneous group of cells, and includes a group of cells derived from adipose tissue, in addition to blood-derived cells. The cells derived from adipose tissue include adipose stromal cells, which include the adipose-derived mesenchymal stem cells (ASCs).

The "mesenchymal stem cell" may be either obtained from an organism or prepared artificially. A composition containing an effective amount of mesenchymal stem cell can also be used in the co-culturing.

The number of days for the co-culturing of the in vitro amplified and cultured mononuclear cell fraction with the mesenchymal stem cell is not particularly limited. In one embodiment, the in vitro amplified and cultured mononuclear cell fraction is co-cultured with the mesenchymal stem cell for a period in the range of from a half day (12 hours) to seven days. In one embodiment, they are co-cultured for a period in the range of from a half day to six days, from a half day to five days, from a half day to four days, from a half day to three days, from one to seven days, from one to six days, from one to five days, from one to four days, or from one to three days.

In one embodiment, the in vitro amplified and cultured mononuclear cell fraction and the mesenchymal stem cell are co-cultured for one to seven days.

The ratios of cells between the in vitro amplified and cultured mononuclear cell fraction and the mesenchymal stem cell to be co-cultured are not particularly limited. In one embodiment, the mesenchymal stem cells are co-cultured with in vitro amplified and cultured mononuclear cells so that the number of mononuclear cells is 0.01 times or more, 0.03 times or more, 0.05 times or more, 0.07 times or more, 0.08 times or more, 0.09 times or more, or 0.1 times or more the number of mesenchymal stem cells. In one embodiment, the mesenchymal stem cell is co-cultured with the in vitro amplified and cultured mononuclear cells so that the number of mononuclear cells is ten times or less, eight times or less, five times or less, four times or less, three times or less, or two times or less the number of mesenchymal stem cells. Taking the amount of blood to be collected into consideration, it is preferable to keep the number of the in vitro amplified and cultured mononuclear cells used low. In one embodiment, the ratio of the number of mesenchymal stem cells to the number of the in vitro amplified and cultured mononuclear cells during the co-culturing is in the range of from 1:0.01 to 1:10. Preferably, it is in the range of from 1:0.05 to 1:4, or from 1:0.1 to 1:2.

The culture medium for the co-culturing is not particularly limited. In one embodiment, a medium suitable for culturing mesenchymal stem cells is used. The medium suitable for culturing mesenchymal stem cells can be appropriately prepared depending on the type of the mesenchymal stem cell. In one embodiment, as a culture medium for adipose-derived mesenchymal stem cells (ASCs), an ASC medium (for example, DMEM (high glucose) + 10% FBS + 1% penicillin/streptomycin) can be used, for example. Alternatively, a differentiation-inducing medium suitable for inducing differentiation of the mesenchymal stem cells into somatic cells can also be used. For example, an adipogenic differentiation inducing medium which induces differentiation of adipose-derived mesenchymal stem cells into adipocytes (for example, DMEM + 10%FBS + insulin +) may be used.

Alternatively, a medium suitable for culturing the in vitro amplified and cultured mononuclear cells may be used. As a culture medium for the in vitro amplified and cultured mononuclear cells, for example, IMDM + 10% FBS can be used.

The composition containing the in vitro amplified and cultured mononuclear cell fraction may be, after being co-cultured with the mesenchymal stem cell, used for differentiation of the mesenchymal stem cell into an adipocyte, as it is.

Alternatively, the composition containing the in vitro amplified and cultured mononuclear cell fraction may be, after being co-cultured with the mesenchymal stem cell, separated from the mesenchymal stem cell during differentiation of the mesenchymal stem cell into an adipocyte (including before performing differentiation induction of the mesenchymal stem cell into an adipocyte and during performing the differentiation induction of the mesenchymal stem cell into an adipocyte). The composition containing the in vitro amplified and cultured mononuclear cell fraction after being used for the co-culturing with the mesenchymal stem cell, for example, may be separated from the mesenchymal stem cell either before performing differentiation induction of the mesenchymal stem cell into an adipocyte or after starting the differentiation induction of the mesenchymal stem cell into an adipocyte (i.e., in the middle of the differentiation induction).

If the composition containing the in vitro amplified and cultured mononuclear cell fraction, after being co-cultured with the mesenchymal stem cell, is separated from the mesenchymal stem cell, the composition used for differentiation of the mesenchymal stem cell into an adipocyte after the separation only contains the mesenchymal stem cell. That is, the composition containing the in vitro amplified and cultured mononuclear cell fraction amplifies the ability of the mesenchymal stem cell to differentiate into an adipocyte, and this effect may be sustained even after the in vitro amplified and cultured mononuclear cell fraction is removed.

The method for separating the composition containing the in vitro amplified and cultured mononuclear cell fraction, after being co-cultured with the mesenchymal stem cell, from the mesenchymal stem cell during differentiation of the mesenchymal stem cell into an adipocyte is not particularly limited. For example, during the co-culturing, an insert may be used in which the mesenchymal stem cells and the in vitro amplified and cultured mononuclear cell fraction are separated by a membrane through which the cells cannot pass but the culture can pass. By culturing only the in vitro amplified and cultured mononuclear cell fraction in the insert and removing the insert after the co-culturing, the composition containing the in vitro amplified and cultured mononuclear cell fraction can be separated from the mesenchymal stem cells. As such a membrane, for example, a polyethylene terephthalate membrane with the pore size of 0.4 $\mu$m and the pore density of $2.0 \times 10^6$ pores/cm$^2$ can be used.

Alternatively, the separation may be performed without using the insert, by directly co-culturing the in vitro amplified and cultured mononuclear cell fraction with the mesenchymal stem cells and then sorting only the mesenchymal stem cells using a flow cytometer.

By the co-culturing with the composition containing the in vitro amplified and cultured mononuclear cell fraction, the ability of the mesenchymal stem cell to differentiate into an adipocyte is amplified.

The term "ability of a mesenchymal stem cell to differentiate into an adipocyte" refers to the capability of differentiating from the mesenchymal stem cell into an adipocyte. The term "amplification of ability of a mesenchymal stem cell to differentiate into an adipocyte" means that differentiation of the mesenchymal stem cell into an adipocyte is enhanced when the mesenchymal stem cell is co-cultured with the composition containing the in vitro amplified and cultured mononuclear cell fraction, as compared to the case without the co-culturing.

"Differentiation into an adipocyte" can be indicated, for example, by expression of an adipocyte marker, an adipogenic differentiation related gene, or the like. Examples of the adipocyte marker include FABP4, Adiponectin, and Perilipin. Examples of the adipogenic differentiation related gene include PPAR$\gamma$, C/EBP$\alpha$, and AP-1. In one embodiment, the term "amplification of ability of a mesenchymal stem cell to differentiate into an adipocyte" means that the expression of at least one of an adipocyte marker, an adipogenic differentiation related gene, or the like is increased, preferably by 5% or more, 10% or more, 20% or more, 30% or more, 40%, or 50% or more, when the mesenchymal stem cell is co-cultured with the composition containing the in vitro amplified and cultured mononuclear cell fraction, as compared to the case without the co-culturing.

Alternatively, the "differentiation into an adipocyte" can be evaluated by staining a component characteristic of adipocytes, such as lipids, to identify adipocytes. For example, Oil Red O has low polarity and thus dissolves specifically in oil droplet to stain lipids red. Therefore, it can be used to evaluate the degree of differentiation of adipocytes (Oil Red O staining).

Note that WO2019/146131 (PTL 3) does not mention at all to the differentiation of adipose-derived mesenchymal stem cells (ASCs) into adipocytes. The enhancement of the angiogenesis capability and the enhancement of adipogenic differentiation are caused by completely different mechanisms, and it is impossible to assume the capability of enhancing adipogenic differentiation from the description in WO2019/146131.

It is desirable to perform in vitro or ex vivo the co-culturing with the composition containing the in vitro amplified and cultured mononuclear cell fraction. The mesenchymal stem cell with amplified "ability to differentiate into an adipocyte" which is obtained by the co-culturing can be used, for example, in grafting for adipose tissue regeneration. In one embodiment, either simultaneously with or after the co-culturing of the mesenchymal cell and the composition containing the in vitro amplified and cultured mononuclear cell fraction, the mesenchymal stem cell cultured in an adipogenic differentiation inducing medium which induces differentiation of adipocytes may be used in grafting. The adipogenic differentiation inducing medium which induces differentiation of mesenchymal stem cells into adipocytes contains, for example, "DMEM + 10%FBS + insulin."

The mesenchymal stem cells with amplified ability to differentiate into adipocytes are applied in an appropriate manner for treatment, such as grafting, to a subject, such as a human. The subject of the treatment is not particularly limited, as long as it is a subject requiring adipose tissue regeneration, and includes mammals, such as human, non-human primates, bovine, equine, porcine, ovine, caprine, canine, feline, and rodents (mouse, rat, and the like). Preferably, the subject is a human. The mesenchymal stem cells can be applied (used) to the subject by a medical professional (such as a doctor and a veterinarian) in an appropriate dosage and administration, depending on the treatment site, the severity of the disease/condition, and the conditions of the subject (age, body weight, height, medical history, physical condition, and the like).

If the composition containing the in vitro amplified and cultured mononuclear cell fraction is separated from the mesenchymal stem cell after the co-culturing with the mesenchymal stem cell, when the grafting is performed, the composition not containing the mononuclear cell fraction but containing only the mesenchymal cells which can be grafted allogeneically is used. Thus, the mononuclear cell fraction is not limited to be derived autologously. Therefore, for both the mononuclear cell fraction and the mesenchymal stem cells, cells which are not derived from a patient but derived from a healthy human can be used. As a result, it is possible to produce a cell preparation and a pharmaceutical composition and administer them to a patient without subjecting the patient to an invasive procedure of cell collection. In addition, for preparation of the composition used in the treatment, since it can be produced and get ready before the treatment, it can be administered to the patient quickly at any time. Furthermore, by preparing the composition in advance and cryopreserving it for a long period of time, advantageous effects can be achieved, such as that it is possible to perform the treatment more quickly with reduced burden on the patient.

2. Method for Amplifying Ability of Mesenchymal Stem Cell to Differentiate into Adipocyte

The present invention also relates to a method for amplifying ability of a mesenchymal stem cell to differentiate into an adipocyte. In one embodiment, the method of the present invention includes co-culturing in vitro amplified and cultured mononuclear cell fraction with a mesenchymal stem cell. In one embodiment, the method is an in vitro or ex vivo method.

Non-limitingly, the method further includes separating the in vitro amplified and cultured mononuclear cell fraction from the mesenchymal stem cell.

The meanings of the terms "in vitro amplified and cultured mononuclear cell fraction," "mesenchymal stem cell," "co-culturing," "amplification of ability to differentiate into an adipocyte," "separating from in vitro amplified and cultured mononuclear cell fraction" and the like, are the same as in "1. Composition for Amplifying Ability of Mesenchymal Stem Cell to Differentiate into Adipocyte." The matters explained in "1. Composition for Amplifying Ability of Mesenchymal Stem Cell to Differentiate into Adipocyte" are similarly applied to this section of "Method for Amplifying Ability of Mesenchymal Stem Cell to Differentiate into Adipocyte," unless particular contradiction occurs.

3. Use and the Like

The present invention also relates to use of in vitro amplified and cultured mononuclear cell fraction for amplifying ability of a mesenchymal stem cell to differentiate into an adipocyte, wherein the in vitro amplified and cultured mononuclear cell fraction is co-cultured with a mesenchymal stem cell.

In one embodiment, the in vitro amplified and cultured mononuclear cell fraction may be, after being co-cultured with the mesenchymal stem cell, separated from the mesenchymal stem cell during differentiation of the mesenchymal stem cell into an adipocyte (including before performing differentiation induction of the mesenchymal stem cell into an adipocyte and during performing the differentiation induction of the mesenchymal stem cell into an adipocyte).

The present invention also relates to in vitro amplified and cultured mononuclear cell fraction for amplifying ability of a mesenchymal stem cell to differentiate into an adipocyte, wherein the in vitro amplified and cultured mononuclear cell fraction is co-cultured with a mesenchymal stem cell. In one embodiment, the in vitro amplified and cultured mononuclear cell fraction may be, after being co-cultured with the mesenchymal stem cell, separated from the mesenchymal stem cell during differentiation of the mesenchymal stem cell into an adipocyte (including before performing differentiation induction of the mesenchymal stem cell into an adipocyte and during performing the differentiation induction of the mesenchymal stem cell into an adipocyte).

The present invention also relates to a method for producing a mesenchymal stem cell with amplified ability to differentiate into an adipocyte, the method comprising co-culturing in vitro amplified and cultured mononuclear cell fraction with a mesenchymal stem cell.

The present invention also relates to a composition containing a mesenchymal stem cell with amplified ability to differentiate into an adipocyte, which is produced by co-culturing in vitro amplified and cultured mononuclear cell fraction with the mesenchymal stem cell. In one embodiment, the in vitro amplified and cultured mononuclear cell fraction may be, after being co-cultured with the mesenchymal stem cell, separated from the mesenchymal stem cell during differentiation of the mesenchymal stem cell into an adipocyte (including before performing differentiation induction of the mesenchymal stem cell into an adipocyte and during performing the differentiation induction of the mesenchymal stem cell into an adipocyte).

The meanings of the terms "in vitro amplified and cultured mononuclear cell fraction," "mesenchymal stem cell," "co-culturing," "amplification of ability to differentiate into an adipocyte," "separating from in vitro amplified and cultured mononuclear cell fraction" and the like, are the same as in "1. Composition for Amplifying Ability of Mesenchymal Stem Cell to Differentiate into Adipocyte" and "2. Method for Amplifying Ability of Mesenchymal Stem Cell to Differentiate into Adipocyte." The matters explained in "1. Composition for Amplifying Ability of Mesenchymal Stem Cell to Differentiate into Adipocyte" and "2. Method for Amplifying Ability of Mesenchymal Stem Cell to Differentiate into Adipocyte" are similarly applied to this section of "Use and the Like," unless particular contradiction occurs.

EXAMPLES

Hereinafter, the present invention will be described in detail with reference to Examples, but the present invention is not limited to the Examples. Modifications and variations can be easily added to the present invention by those skilled in the art based on the description herein, and are encompassed within the technical scope of the present invention.

Materials and Methods

(1) Preparation of Adipose-derived Mesenchymal Stem Cells (ASCs)

Human adipose tissue used was abdominal adipose tissue disposed of during a surgery in clinical studies approved by the Ethics Committee of Juntendo University.

After washing with PBS, the adipose tissue was digested with an equal amount of 0.1% collagenase 1 solution (WAKO PURE CHEMICAL). An equal amount of ASC medium (DMEM (high glucose, Invitrogen) + 10% FBS + 1% penicillin/streptomycin) was added to quench the enzymatic reaction. After centrifugal separation, the pellets were filtrated using a 100 $\mu$m cell strainer (manufactured by Corning). The residue was washed with the ASC medium, and then disseminated and cultured in a flask. The fifth passage ASCs were subjected to flow cytometry (BD LSRFortessa, BD Biosciences) to measure the expression of surface markers on the cells. Cells confirmed for being positive for CD73 and CD90 and negative for CD31, CD34, CD45, and HLA-DR, which is the characteristic of ASCs, were used in the Examples.

(2) Preparation of RE01 Cells

(i) Isolation of Mononuclear Cells

Using a BD Vacutainer (registered trademark) CPT (trademark) (manufactured by BD) blood collection tube for mononuclear cell separation, 8 mL of peripheral blood was collected from healthy human volunteers. After collecting the blood, the blood collection tube was centrifuged as it was, then transported, and stored under refrigeration after the arrival and until culturing was started. The part above the gel barrier in the CPT (trademark) blood collection tube containing mononuclear cells and plasma was collected into a centrifugation tube, and the inside of the blood collection tube, including above the gel barrier, was washed with a small amount of EDTA-PBS, and the cells were collected into the same centrifugation tube. The centrifugation tube into which the cells had been collected was filled up to the gauge line with EDTA-PBS, and then was subjected to centrifugal separation ($300 \times g$, room temperature, 10 minutes) to collect the cells as sediment. The collected cells were incubated for five minutes at room temperature in an ACK hemolysis buffer (15 mL/tube) (Gibco, manufactured by Thermo-Fisher) in order to remove contaminating red blood cells. The composition of the ACK hemolysis buffer is as follows: 8,290 mg/l of $NH_4Cl$; 1,000 mg/l of $KHCO_3$; and 37 mg/l of $EDTA.Na_2 \cdot 2H_2O$. The cells were then diluted with EDTA-PBS up to the gauge line and were subjected to centrifugal separation (($200 \times g$, room temperature, 10 minutes) or ($100 \times g$, room temperature, 15 minutes)) to collect the cells. This procedure was repeated two times.

(ii) Culture in Serum Medium

The collected mononuclear cells were suspended in a medium containing an IMDM (including GlutaMAX, Gibco) supplemented with 50 ng/mL of VEGF, 100 ng/mL of FLT-3 ligand, 20 ng/mL of TPO, 0.5% FBS, 0.5% HSA (Sekijuji Albumin 25%, Japan Blood Products Organization), 100 units/mL of penicillin, and 100 $\mu$g/mL of streptomycin. A portion of the suspension was used for cell counting using trypan blue. In the resulting cell proliferation medium, the cells were prepared to have a cell concentration of $1 \times 10^6$/mL, disseminated in a T25 flask, and were cultured for five days under normal culture conditions (37°C, 5% $CO_2$).

After the culturing for five days was finished, the culture medium was collected in a centrifugation tube, and centrifugal washing was performed three times for seven to ten minutes at $250 \times g$ (about 1000 rpm). After the centrifugation, the cells were suspended in PBS. The resulting group of cells may be herein referred to as "RE-01."

Example 1. Induction of Adipogenic Differentiation (RE01 Cells are Not Separated after Co-culturing)

(1) Co-culturing

The ASCs prepared as in the section (1) of "Materials and Methods" above were disseminated in a 6-well plate or a 24-well plate at $7 \times 10^4$ cells/well, and cultured in an ASC medium (DMEM (high glucose), manufactured by Invitrogen) + 10% FBS + 1% penicillin/streptomycin) for one night.

An insert (manufactured by Corning) was then set, and the RE01 cells prepared as in the section (2) above were disseminated so that the number of cells was 1/2 of ASC (ASC + RE01 2: 1) or 1/10 (ASC + RE01 10: 1).

The insert and the culture plate are separated by a membrane with a pore size of 0.4 $\mu$m, and the cells cannot pass through the membrane while the culture can pass through the membrane. In a state in which the insert with the disseminated RE01 cells had been set in the culture plate with the disseminated ASCs, culturing was performed in an adipogenic differentiation inducing medium (DMEM + 10% FBS + insulin +) at 37°C and under 5% $CO_2$. After three days from the initiation of the culturing, RNAs were collected for performing an mRNA analysis. Furthermore, after two weeks from the initiation of the culturing, lipid droplets were stained with Oil Red O staining.

(2) mRNA Analysis

As mRNA analysis, mRNA expressions of FABP4 which is an adipocyte marker, and PPAR$\gamma$ which is an adipogenic differentiation related gene were analyzed. Specifically, RNAs after co-culturing the ASCs and the RE01 cells (with induction of adipogenic differentiation performed simultaneously) for three days were purified, and cDNAs were synthesized. The resulting cDNAs were subjected to real-time PCR for measuring and analyzing expression levels of respective genes, that is, FABP4 and PPAR$\gamma$.

After the co-culturing in the Example 1, only the ASCs were separated and washed with PBS, and then the Total RNAs were extracted using RNeasy Micro Kit (Qiagen). From 1 to 2 $\mu$g of RNAs, cDNAs were synthesized using High-Capacity RNA-to-cDNA Kit (Applied Biosystems). Then reagents were adjusted using TaqMan fast advanced master mix (Applied Biosystems), and measurement and analysis were performed using StepOne Plus (Applied Biosystems).

All of the TaqMan probes used for the analysis were purchased from Applied Biosystems:

FABP4 (Hs01086177_m1);
PPARγ (Hs01115513_m1); and
18S-rRNA (Hs03928990_g1).

Using the ΔΔCt method (comparative Ct method), the mRNA expressions were analyzed. In the ΔΔCt method, comparison is performed by the following three calculations:

$$\Delta Ct = (\text{target gene Ct}) - (\text{internal control Ct});$$

$$\Delta\Delta Ct = (\text{each sample } \Delta Ct) - (\text{reference sample average } \Delta Ct^0); \text{ }$$

and
plugging the obtained ΔΔCt into 2-(-ΔΔCt) and comparing the numbers.

As the internal control Ct, the value of 18S-rRNA was used.

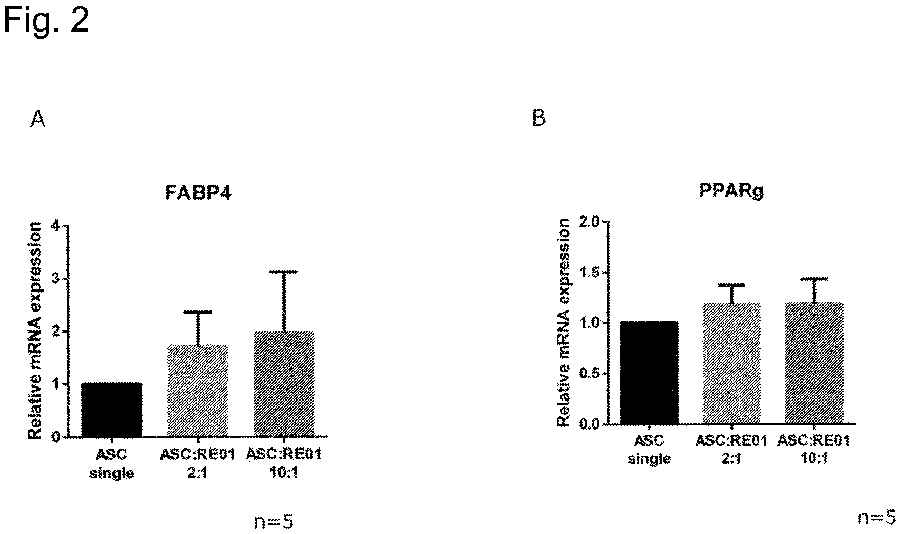

The results of the mRNA analysis are illustrated in Fig. 2. The ASCs co-cultured with the RE01 cells tended to have increased mRNA expression levels of FABP4 and PPARγ.

(3) Staining with Oil Red O

After co-culturing the ASCs and the RE01 cells (with induction of adipogenic differentiation performed simultaneously) for two weeks, only the ASCs were separated and washed with PBS three times, and then immobilized with 4% PFA for one hour. After washing three times with distilled water, the cells were dehydrated with 60% isopropanol and stained with Oil Red O staining solution (Muto Chemicals) for 20 minutes. Subsequently, the resultant was washed three times with distilled water, and the nuclei were stained with Mayer's hematoxylin (Fujifilm Wako Pure Chemicals) for five minutes. After washing with distilled water, the resultant was photographed under a microscope (BZ-X710, Keyence).

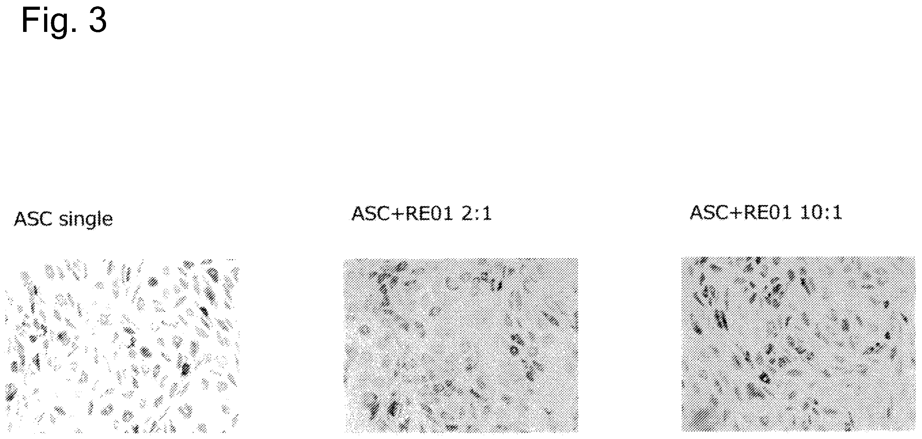

The results are illustrated in Fig. 3. It was observed that the ASCs co-cultured with RE01 cells tended to have a higher number of adipocytes stained red by Oil Red O staining. It indicates that the co-culturing with RE01 cells tends to enhance the adipogenic differentiation capability of the ASCs.

Example 2. Induction of Adipogenic Differentiation (RE01 Cells are Separated after Co-culturing)

The ASCs prepared as in the section (1) of "Materials and Methods" above were disseminated in a 6-well plate or a 24-well plate at $7 \times 10^4$ cells/well, and cultured in an ASC medium (DMEM (high glucose), manufactured by Invitrogen) + 10% FBS + 1% penicillin/streptomycin) for one night.

An insert (manufactured by Corning) was then set, and the RE01 cells prepared as in the section (2) above were disseminated on the insert so that the number of cells was 1/2 of ASC (ASC + RE01 2: 1) or 1/10 (ASC + RE01 10:1).

The insert and the culture plate are separated by a membrane with a pore size of 0.4 μm, and the cells cannot pass through the membrane while the culture can pass through the membrane. In a state in which the insert with the disseminated RE01 cells had been set in the culture plate with the disseminated ASCs, culturing was performed in an ASC medium for three days at 37°C and under 5% $CO_2$. From the co-cultured product (after three days of co-culturing) of the ASCs and the RE01 cells, the insert (RE01 cells) was removed, and only the ASCs in the 6-well culturing plate were separated.

Subsequently, the separated ASCs were cultured in an adipogenic differentiation inducing medium (DMEM + 10% FBS + insulin +) at 37°C and under 5% $CO_2$. After three days from the initiation of the induction of adipogenic differentiation, RNAs were collected for performing an mRNA analysis. Furthermore, after two weeks from the initiation of the induction of adipogenic differentiation, lipid droplets were stained with Oil Red O staining.

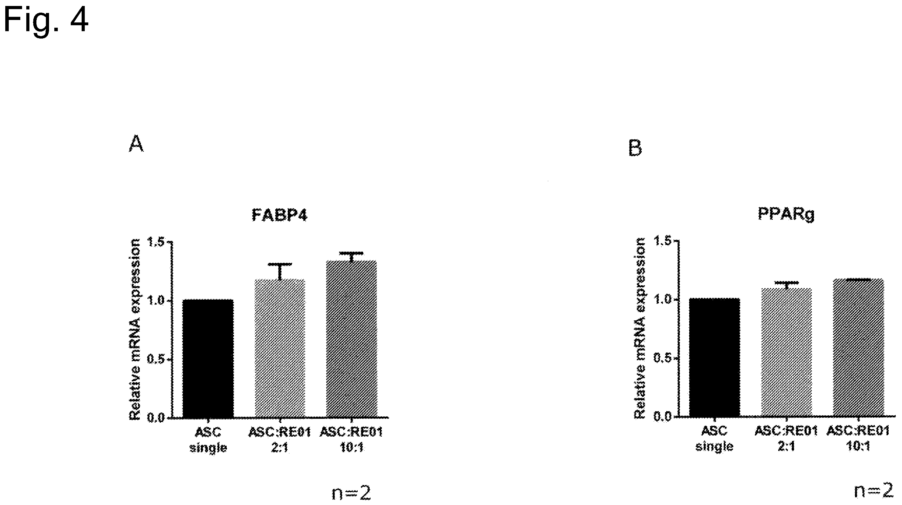

The mRNA analysis was conducted as in the Example 1. The results are illustrated in Fig. 4. The ASCs co-cultured with the RE01 cells tended to have increased mRNA expression levels of FABP4 and PPARγ, also in the case where induction of adipogenic differentiation was performed for ASCs alone after separation from the RE01 cells.

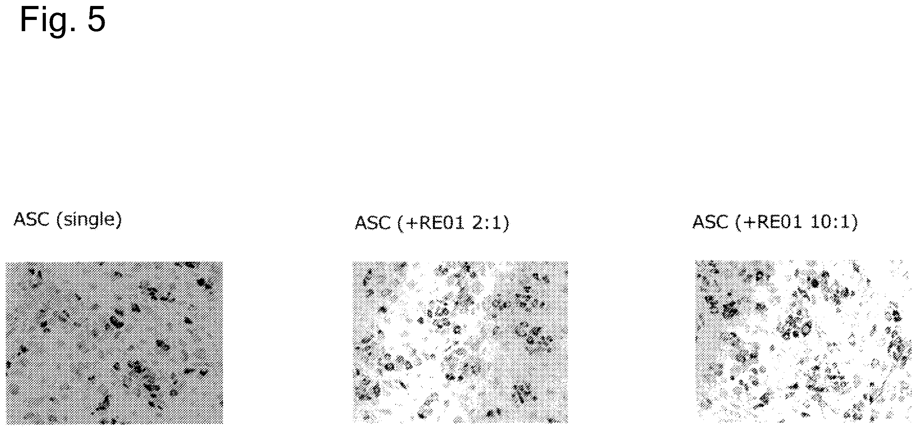

The staining with Oil Red O was conducted as in the Example 1. The results are illustrated in Fig. 5. It was observed that the ASCs co-cultured with RE01 cells tended to have a higher number of adipocytes stained red by Oil Red O staining, also in the case where induction of adipogenic differentiation was performed for ASCs alone after separation from the RE01 cells. The ASCs co-cultured with the RE01 cells maintains the state with enhanced adipogenic differentiation capability, also when they are ASCs alone after separation from the RE01.

INDUSTRIAL APPLICABILITY

The Examples herein demonstrate that ASCs co-cultured with RE01 cells have enhanced adipogenic differentiation capability, as compared with ASCs not co-cultured with RE01 cells. The present invention enables to provide a pharmaceutical product with higher adipogenic differentiation capability than pharmaceutical products containing ASCs not co-cultured with RE01 cells (that is, existing pharmaceutical products containing mesenchymal stem cells).

**Claims**

1.   A composition for amplifying ability of a mesenchymal stem cell to differentiate into an adipocyte, wherein the composition comprises in vitro amplified and cultured mononuclear cell fraction and is co-cultured with a mesenchymal stem cell.

2.   The composition according to claim 1, wherein the composition is separated from the mesenchymal stem cell during differentiation of the mesenchymal stem cell into an adipocyte.

3.   The composition according to claim 1, wherein the in vitro amplified and cultured mononuclear cell fraction is co-cultured with a mesenchymal stem cell for one to seven days.

4.   The composition according to claim 1 or 2, wherein the mononuclear cell fraction is a mononuclear cell fraction derived from bone marrow, peripheral blood, or umbilical cord blood.

5.   The composition according to claim 1 or 2, wherein the in vitro amplified and cultured mononuclear cell fraction is a group of cells rich in endothelial progenitor cells or anti-inflammatory cells or immunotolerance-inducing cells.

6.   The composition according to claim 1 or 2, wherein the mesenchymal stem cell is an adipose-derived mesenchymal stem cell, a bone marrow-derived mesenchymal stem cell, a peripheral blood-derived mesenchymal stem cell, or an umbilical cord blood-derived mesenchymal stem cell.

7.   A method for amplifying ability of a mesenchymal stem cell to differentiate into an adipocyte, the method comprising co-culturing in vitro amplified and cultured mononuclear cell fraction with a mesenchymal stem cell.

8.   The method according to claim 7, further comprising separating the in vitro amplified and cultured mononuclear cell fraction from the mesenchymal stem cell.

9.   A method for producing a mesenchymal stem cell with amplified ability to differentiate into an adipocyte, the method comprising co-culturing in vitro amplified and cultured mononuclear cell fraction with a mesenchymal stem cell.

10.  A composition comprising a mesenchymal stem cell with amplified ability to differentiate into an adipocyte, wherein the mesenchymal stem cell is produced by co-culturing in vitro amplified and cultured mononuclear cell fraction with a mesenchymal stem cell.

Fig. 1

Fig. 2

A

FABP4

ASC single | ASC:RE01 2:1 | ASC:RE01 10:1

Relative mRNA expression

n=5

B

PPARg

ASC single | ASC:RE01 2:1 | ASC:RE01 10:1

Relative mRNA expression

n=5

Fig. 3

Fig. 4

A

B

# Fig. 5

EP 4 636 077 A1

ASC (single)

ASC (+RE01 2:1)

ASC (+RE01 10:1)

## INTERNATIONAL SEARCH REPORT

| | International application No. |
|---|---|
| | **PCT/JP2023/044432** |

| **A.** | **CLASSIFICATION OF SUBJECT MATTER** |
|---|---|

*C12N 5/077*(2010.01)i; *C12N 5/078*(2010.01)i; *C12N 5/0775*(2010.01)i
FI:   C12N5/077; C12N5/0775; C12N5/078

According to International Patent Classification (IPC) or to both national classification and IPC

| **B.** | **FIELDS SEARCHED** |
|---|---|

Minimum documentation searched (classification system followed by classification symbols)

C12N5/077; C12N5/078; C12N5/0775

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| A | 古川聖美他. 乳房再建にMNC-QQ細胞を用いた新・脂肪移植技術の開発. 第30回日本形成外科学会基礎学術集会　プログラム・要旨集. 2021, p. 66, SY3-1, non-official translation (FURUKAWA, Satomi et al. Development of Novel Fat Grafting Technique Using MNC-QQ Cells for Breast Reconstruction. Program and Abstracts of the 30th Research Council Meeting of the Japan Society of Plastic and Reconstructive Surgery.) Background, Results | 1-10 |
| A | WO 2021/131261 A1 (JUNTENDO UNIVERSITY) 01 July 2021 (2021-07-01) claims, examples | 1-10 |
| A | WO 2019/146131 A1 (JUNTENDO UNIVERSITY) 01 August 2019 (2019-08-01) claims, examples | 1-10 |
| A | JP 2017-93431 A (AWS HOLDINGS INC.) 01 June 2017 (2017-06-01) claims 1, 12 | 1-10 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 February 2024** | **27 February 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

International application No.

**PCT/JP2023/044432**

| Patent document cited in search report | | | Publication date (day/month/year) | Patent family member(s) | | | Publication date (day/month/year) |
|---|---|---|---|---|---|---|---|
| WO | 2021/131261 | A1 | 01 July 2021 | US | 2023/0034582 | A1 | |
| | | | | claims, examples | | | |
| | | | | EP | 4082613 | A1 | |
| | | | | CN | 114901806 | A | |
| WO | 2019/146131 | A1 | 01 August 2019 | (Family: none) | | | |
| JP | 2017-93431 | A | 01 June 2017 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019146131 A
- WO 2006090882 A
- WO 20140561154 A
- WO 2021131261 A

**Non-patent literature cited in the description**

- *Plast Reconstr Surg.,* February 2013, vol. 131 (2), 185-191
- *Langenbecks Arch Klin Chir Ver Dtsch Z Chir.*, 1893, vol. 22, 66-69
- *Otolaryngol Head Neck Surg.*, April 1990, vol. 102 (4), 314-321
- *Ann Plast Surg.*, July 2005, vol. 55 (1), 63-68
- *Exp Biol Med (Maywood).*, November 2005, vol. 230 (10), 742-748
- *Dermatol Surg.*, December 2006, vol. 32 (12), 1437-1443
- *Plast Reconstr Surg Glob Open.*, 07 October 2013, vol. 1 (6), e40
- *Plast Reconstr Surg.*, May 2012, vol. 129 (5), 1081-1092
- *Plast Reconstr Surg.*, June 2015, vol. 135 (6), 1607-1617
- *J Am Coll Cardiol.*, 17 December 2003, vol. 42 (12), 2073-2080
- *Plast Reconstr Surg.*, June 2008, vol. 121 (6), 1929-1942
- *Stem Cells Transl Med.*, February 2012, vol. 1 (2), 160-171
- *Mol Biol Cell*, December 2002, vol. 13 (12), 4279-4295
- *J Plast Reconstr Aesthet Surg.*, November 2013, vol. 66 (11), 1494-1503
- *Aesthet Surg J.*, 01 July 2017, vol. 37 (3), 46-58
- *Stem Cells.*, January 2009, vol. 27 (1), 266-274
- *Circulation*, 10 February 2004, vol. 109 (5), 656-663
- *PLoS One.*, 2012, vol. 7 (9), e45621
- *Lancet.*, 28 September 2013, vol. 382 (9898), 1113-1120
- *Diabetes.*, September 2013, vol. 62 (9), 3207-3217
- *J Am Heart Assoc.*, 25 June 2014, vol. 3 (3), e000743
- *Japanese Journal of Thrombosis and Hemostasis*, 2014, vol. 25 (5), 593-602